# EUROPÄISCHE PATENTSCHRIFT

(11) **EP 3 334 616 B1**
(45) Veröffentlichungstag und Bekanntmachung des Hinweises auf die Patenterteilung: **11.05.2022**
(21) Anmeldenummer: 16767015.7
(22) Anmeldetag: 11.08.2016
(51) Int. Cl.: B60H 3/00

(54) **KLIMAGERÄT**
AIR-CONDITIONING DEVICE
CLIMATISEUR

(30) Priorität: 12.08.2015 DE 102015113276
(43) Veröffentlichungstag der Anmeldung: 20.06.2018
(73) Patentinhaber: Relyon Plasma GmbH, 93055 Regensburg (DE); TDK Electronics AG, 81671 München (DE)
(72) Erfinder: NETTESHEIM, Stefan, 93051 Regensburg (DE); BURGER, Dominik, 93087 Alteglofsheim (DE); KÜGERL, Georg, 8552 Eibiswald (AT); PUFF, Markus, 8010 Graz (AT)
(74) Vertreter: Reichert & Lindner Partnerschaft Patentanwälte
(86) Internationale Anmeldenummer: PCT/IB2016/054843
(87) Internationale Veröffentlichungsnummer: WO 2017/025923

(56) Entgegenhaltungen:
- WO-A1-2011/061913
- JP-A- 2010 042 750
- JP-A- 2010 149 536
- KR-A- 20060 088 990
- US-A1- 2005 058 582
- US-A1- 2005 169 821

## Beschreibung

Die vorliegende Anmeldung betrifft ein Klimagerät, insbesondere ein Klimagerät für die Verwendung in einem Kraftfahrzeug. Das Klimagerät ist für einen zu klimatisierenden Raumbereich und umfasst ein Gehäuse mit einer Luftzufuhr und einer Luftabfuhr. Zwischen der Luftzufuhr und der Luftabfuhr ist ein Strömungsweg ausgebildet, wobei im Strömungsweg zumindest ein Wärmetauscher, ein Gebläse und eine Einrichtung zur Erzeugung eines angeregten Gases vorgesehen sind.

DE 20 2012 010 239 U1 offenbart eine Vorrichtung zur Luftreinigung. Die Vorrichtung umfasst ein Gehäuse, einen elektrostatischen Aufladeabschnitt und einen Geruchsfilter. Ein Luftstrom wird in einem Gehäuse vom Lufteinlass, zu einem Plasmafilter, der den elektrostatischen Aufladeabschnitt und den Geruchsfilter umfasst, zum Luftauslass weitergeleitet. Der Aufladeabschnitt umfasst wenigstens eine plattenförmige Plasmaelektrode zur Erzeugung des Plasmas.

DE 10 2014 107 805 A1 betrifft zunächst eine Anordnung zur Filterung von Luft, wobei in einem Luftkanal auch eine Plasma-Zelle, ein Katalysator und ein Gebläserad vorgesehen sind.

DE 10 2004 034 432 A1 betrifft einen Filter und/oder eine Filteranordnung für eine Kraftfahrzeug-Klimaanlage und/oder ein Kraftfahrzeug-Belüftungssystem. Der Filter wird von einem, insbesondere durch ein Gebläse angetriebenen, Luftstrom durchströmt, und der Filter wandelt einen oder mehrere Schadstoffe in ungefährliche Stoffe um. Dabei besteht der Filter zumindest teilweise aus einem eiweißreichen Material, das mit einem katalytischen Wirkstoff beschichtet ist.

DE 11 2013 001 665 T5 betrifft eine Vorrichtung zum Reinigen des Klimaanlagensystems von Fahrzeugen. Hierzu wird eine katalytische Strahlungsionisation verwendet. Mittels einer UVX-Glühlampe, die von einer Edelmetalllegierung umgeben ist, wird Luft/Sauerstoff in ein reinigendes Plasma umgewandelt, das Hydroxyl-Radikale und Wasserstoffperoxid enthält.

Die Übersetzung DE 60 304 432 T2 der europäischen Patentschrift EP 1 435 306 offenbart eine Klimaanlage für ein Fahrzeug. Ein Modul der Klimaanlage erlaubt das Filtern gasförmiger Zusammensetzungen, wie beispielsweise flüchtiger organischer Zusammensetzungen und Mikroorganismen, die durch die Luft der Klimaanlage des Klimasystems bewegt werden. Hierzu ist auch ein plasmakatalytisches Entgiftungsmodul vorgesehen, das mit einem elektrostatischen Entgiftungsmodul kombiniert ist.

WO 2011/061913 A1 und JP 2010 042750 A offenbaren ein Klimagerät für einen zu klimatisierenden Raumbereich mit den Merkmalen des Oberbegriffs des Anspruchs 1.

Aufgabe der Erfindung ist, ein Klimagerät zu schaffen, das für einen zu klimatisierenden Raumbereich klimatisierte Luft zu Verfügung stellt, die den Eindruck von frischer Luft vermittelt und schädliche Bestandteile der zu klimatisierenden Luft beseitigt.

Die obige Aufgabe wird durch ein Klimagerät gelöst, das die Merkmale des Patentanspruchs 1 umfasst.

Das erfindungsgemäße Klimagerät für einen zu klimatisierenden Raumbereich, umfasst ein Gehäuse mit einer Luftzufuhr und einer Luftabfuhr. Zwischen der Luftzufuhr und der Luftabfuhr ist ein Strömungsweg für die zu klimatisierende Luft ausgebildet. Im Strömungsweg sind zumindest ein Wärmetauscher und ein Gebläse vorgesehen. Eine Einrichtung zur Erzeugung eines angeregten Gases ist ebenfalls im Strömungsweg des Klimageräts angeordnet. Erfindungsgemäß ist die Einrichtung zur Erzeugung eines angeregten Gases ein Plasmaerzeuger, ein lonisator oder ein Ozongenerator und umfasst einen PDD piezoelektrischen Transformator.

Optional ist eine Oberflächenbeschichtung im Strömungsweg des angeregten Gases vorgesehen, die zum katalytischen Abbau des Ozongehalts des angeregten Gases dient und der Einrichtung zur Erzeugung des angeregten Gases nachgeordnet ist.

Ein piezoelektrischer Transformator (PT) ist eine Bauform eines Resonanztransformators, welcher auf Piezoelektrizität basiert und im Gegensatz zu den herkömmlichen magnetischen Transformatoren ein elektromechanisches System darstellt. Er dient dazu, eine zugeführte elektrische Wechselspannung einer bestimmten Frequenz, welche von den mechanischen Abmessungen des Transformators bestimmt wird, in eine höhere oder niedrigere Wechselspannung umzusetzen.

PDD piezoelektrische Transformatoren erzeugen hohe elektrische Felder über den piezoelektrischen Effekt. Diese Felder sind in der Lage, Gase und Flüssigkeiten durch elektrische Anregung zu ionisieren. An der Sekundärseite des PT erzeugt das elektrische Wechselfeld eine starke Polarisation, Anregung und Ionisation von Atomen und Molekülen. Dieser Prozess erzeugt ein piezoelektrisch gezündetes Mikroplasma, PDD (Piezoelectric Direct Discharge Plasma). PDDs haben Eigenschaften, die den typischen dielektrischen Barriereentladungen (DBD) entsprechen. PDDs können in einem weiten Druckbereich von 0,01 mbar und 2000 mbar gezündet werden.

Parasitäre Entladungserscheinungen am piezoelektrischen Transformator sind unerwünscht, allerdings kann dieser Effekt auch gezielt eingesetzt werden. Bei PDD kann ein Plasma direkt gezündet werden. Ähnlich wie bei einer stillen elektrischen Entladung (DBD: dielectric barrier discharge) kommt es bei ausreichend hohen oszillierenden Feldstärken zu einer kalten Entladung. Aufgrund der hohen Feldinhomogenität und des Frequenzeinflusses kann auch unter atmosphärischen Bedingungen das umgebende Gas ionisiert werden, ohne dass hierzu die absolute Zündspannung unterhalb der Paschen Kurve liegen muss.

Zur Erzeugung von PDD-Plasma sind piezoelektrische Transformatoren vom Typ Rosen besonders geeignet, da dieser Typ hohe Leistungsdichten und sehr hohe Übersetzungsverhältnisse liefert. Transformationsverhältnisse von mehr als 1000 können in der Praxis erreicht werden. Resonanzfrequenzen zwischen 10 kHz bis 500 kHz sind für das Zünden von PDD-Plasma optimal. Wird der Leistungstreiber optimal an die Resonanz und an die Impedanz des piezoelektrischen Transformators angepasst, erfolgt die Konversion in den Entladungsprozess mit hohem Wirkungsgrad im Gesamtsystem.

Ozongeneratoren, die auf PDD beruhen und mit Luft betrieben werden, liefern eine mittlere Ozonkonzentration mit der höchsten Effizienz der bislang bekannten Systeme. Die Gastemperatur im Plasmavolumen liegt bei PDD typischerweise bei einer Umgebungstemperatur von 300+20 K. Elektronendichten von ca. 1014 und 1016 m⁻³ werden erreicht. Damit liefert PDD ein typisches "kaltes" Nichtgleichgewichtsplasma. Diese Eigenschaften von PDD eröffnen vielfältige Anwendungsmöglichkeiten. PDD Geräte werden eingesetzt in der medizinischen Forschung, zur Keimreduktion, Geruchsreduktion und in der Mikrobiologie. Typische Industrieanwendungen umfassen die Oberflächenaktivierung zur Optimierung von Benetzungs- und Hafteigenschaften bei Kunststoffen, beispielsweise bei Druck-, Lackierungs- und Klebprozessen.

Das Klimagerät kann auch eine Einrichtung zur Steigerung der Aktivität/Reaktivität umfassen, die im Strömungsweg, im Gehäuse des Klimageräts, angeordnet ist. Die Einrichtung zur Erzeugung eines angeregten Gases ist zwischen dem Gebläse und der Einrichtung zur Steigerung der Aktivität/Reaktivität vorgesehen. Gemäß einer weiteren Ausführungsform des Klimageräts ist das Gebläse in Strömungsrichtung der Luft der Einrichtung zur Steigerung der Aktivität/Reaktivität vorgeordnet. Eine weitere Möglichkeit ist, dass das Gebläse in Strömungsrichtung der Luft der Einrichtung zur Steigerung der Aktivität/Reaktivität nachgeordnet ist.

Gemäß einer weiteren Ausführungsform des erfindungsgemäßen Klimageräts ist innerhalb des Gehäuses ein Filter vorgesehen, der in Strömungsrichtung der Luft dem Wärmetauscher vorgeordnet ist. Die Einrichtung zur Erzeugung eines angeregten Gases ist dann dem Filter in Strömungsrichtung der Luft vorgeordnet oder dem Filter in Strömungsrichtung der Luft nachgeordnet.

Die Einrichtung zur Steigerung der Aktivität/Reaktivität kann eine Katalysatorstruktur, eine Aktivkohlestruktur oder ein Reaktivfilter sein. Dabei weist die Einrichtung zur Steigerung der Aktivität/Reaktivität eine katalytisch aktive Oberfläche auf, mittels der durch heterogene Oxidation Moleküle und Mikroorganismen abgebaut werden.

Dem Klimagerät ist der Luftzufuhr in das Gehäuse eine Verteilerklappe vorgeordnet, die wahlweise mit einer Umgebungsluft oder einer Innenluft des zu klimatisierenden Raumbereichs in fluider Verbindung ist. Der Verteilerklappe kann ein Filter für Partikel, Schmutz oder Wasser vorgeordnet sein.

Bei dem Klimagerät ist der Luftabfuhr aus dem Gehäuse ein Verteilersystem nachgeschaltet, so dass die klimatisierte Luft in gewünschter Weise im klimatisierenden Raumbereich verteilt werden kann.

Von besonderem Vorteil ist die Verwendung des erfindungsgemäßen Klimageräts für die Klimatisierung des Innenraums von Kraftfahrzeugen. Durch die geeignete Positionierung von einem Plasmaerzeuger, einem lonisator oder Ozongenerator über elektrische Gasentladung im Strömungsweg der Luft in der Klimaanlage für ein Kraftfahrzeug, kann ein reaktives Gas oder Gasgemisch erzeugt werden, das zusammen mit anderen Komponenten der Klimaanlage für eine Versorgung des Fahrgastinnenraums von Kraftfahrzeugen mit frischer von Gerüchen unbelasteter und von schädlichen Molekülen oder Mikroorganismen befreiter Luft sorgt. Eine der Komponenten ist beispielsweise das Filterelement, das bereits reaktive Bestandteile oder katalytisch aktive Bestandteile enthalten kann, um den Ozongehalt der Luft in der Klimaanlage zu reduzieren. Ebenso kann der Wärmetauscher eine Oberflächenbeschichtung aufweisen, die den Ozongehalt der Luft im Strömungsweg katalytisch abbaut. Der Abbau von schädlichen Gasspezies (Molekülen, Mikroorganismen) in der Luftströmung im Gehäuse des Klimageräts kann durch heterogene Oxidation auf den katalytisch aktiven Oberflächen erfolgen. Mit all diesen Maßnahmen erreicht man eine Zufuhr von klimatisierter Luft in den Innenraum eines Kraftfahrzeugs, die im Wesentlichen sauber und den Eindruck von frischer Luft vermittelt.

Die Einrichtung zur Steigerung der Aktivität/Reaktivität ist gemäß einer Ausführungsform mit Aktivkohle versehen, welche die Aktivkohlestruktur bildet. Die Aktivkohle kann beispielsweise imprägniert sein, wie beispielsweise mit Übergangsmetallen, Platingruppenmetallen und/oder Manganoxid. Aktivkohle oder Manganoxid kann beispielsweise auch als Schichtmaterial auf beliebige Oberflächen aufgetragen werden. In einer Ausführungsform ist die Katalysatorstruktur der Einrichtung zur Steigerung der Aktivität/Reaktivität auf einem Kühler (Kondensator) ausgebildet und besitzt eine hydrophobe Ausstattung, um auch unter feuchten Bedingungen eine hohe aktive Oberfläche zu erhalten (analog zu einer PEM Brennstoffzelle). Diese Oberflächen können beispielsweise am Wärmetauscher, am Gebläse/Verdampfergebläse und an den Innenwandungen der Luftverteilerkanäle beziehungsweise am Verteilersystem ausgebildet sein.

Ein konventioneller Wärmetauscher kühlt typischerweise die zu klimatisierende Luft auf circa 5°C herunter und trocknet damit die Luft auf 5°C Taupunkt. Dieser kalte und trockene Zustand der Luft ist besonders geeignet, um mit der Einrichtung zur Erzeugung eines angeregten Gases hohe Ozonkonzentrationen mit hohem Wirkungsgrad zu erzeugen, begünstigt durch eine geringe Temperatur und eine geringe Feuchte. Im Allgemeinen wird die nach dem Kühlprozess mittels des Kühlerteils (Verdampfers) des Wärmetauschers gekühlte und getrocknete Luft wieder auf circa 20°C, beispielsweise mittels einer geeigneten elektrischen Struktur, geheizt. Diese geheizte Struktur kann katalytisch besonders aktiv sein. Der Wärmetauscher umfasst in seiner Gesamtheit den Kühler (Verdampfer/Kondensator) und einen (Zusatz-)Heizer (beispielsweise geheizte Struktur) und weist eine sehr große Oberfläche auf, die für eine Oberflächenbeschichtung im Sinne der Erfindung geeignet ist.

Allerdings ist bei der obigen Oberflächenbeschichtung problematisch, wenn diese auf eine Oberfläche des Verdampfers aufgebracht ist, dass dort Flüssigkeit auskondensiert. Die Oberfläche wird somit nass und die Aktivität des Katalysators sinkt oder ist sogar nicht mehr vorhanden. Um dies zu vermeiden, sollte die Katalysatorbeschichtung bevorzugt mit einer hydrophoben Beimischung versehen sein, beispielsweise mit Teflon und/oder andere hydrophoben Materialien. Nach dem Verdampfungsprozess mittels des Verdampfers sind die Oberflächen wieder trocken. Zudem können weitere Oberflächen katalytisch ausgerüstet werden, beispielsweise Wände der gesamten Strömungsführung (Verteilersystem), oder insbesondere dort, wo hohe Turbulenzen entstehen, also beispielsweise beim Gebläse. Die Oberfläche des (Zusatz-)Heizers ist im Betrieb erwärmt, wodurch ein dort angebrachter Katalysator eine hohe Aktivität bewirken würde.

Nachfolgend werden die Erfindung und ihre Vorteile unter Bezugnahme auf die beigefügten Zeichnungen ausführlicher beschrieben. Es zeigen:
**Figur 1** eine schematische Ansicht einer Ausführungsform des erfindungsgemäßen Klimageräts;
**Figur 2** eine schematische Ansicht einer weiteren Ausführungsform des erfindungsgemäßen Klimageräts;
**Figur 3** eine schematische Ansicht einer noch weiteren Ausführungsform des erfindungsgemäßen Klimageräts;
**Figur 4** eine schematische Ansicht einer anderen Ausführungsform des erfindungsgemäßen Klimageräts;
**Figur 5** eine schematische Ansicht einer anderen Ausführungsform des erfindungsgemäßen Klimageräts;
**Figur 6** eine schematische Ansicht einer zusätzlich anderen Ausführungsform des erfindungsgemäßen Klimageräts; und
**Figur 7** eine schematische Darstellung der Verwendung des erfindungsgemäßen Klimageräts bei einem Kraftfahrzeug.

In den Figuren sind für gleiche oder gleich wirkende Elemente der Erfindung identische Bezugszeichen verwendet. Ferner sind der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie das erfindungsgemäße Klimagerät ausgestaltet sein kann und ist nicht als abschließende Beschränkung zu verstehen. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.

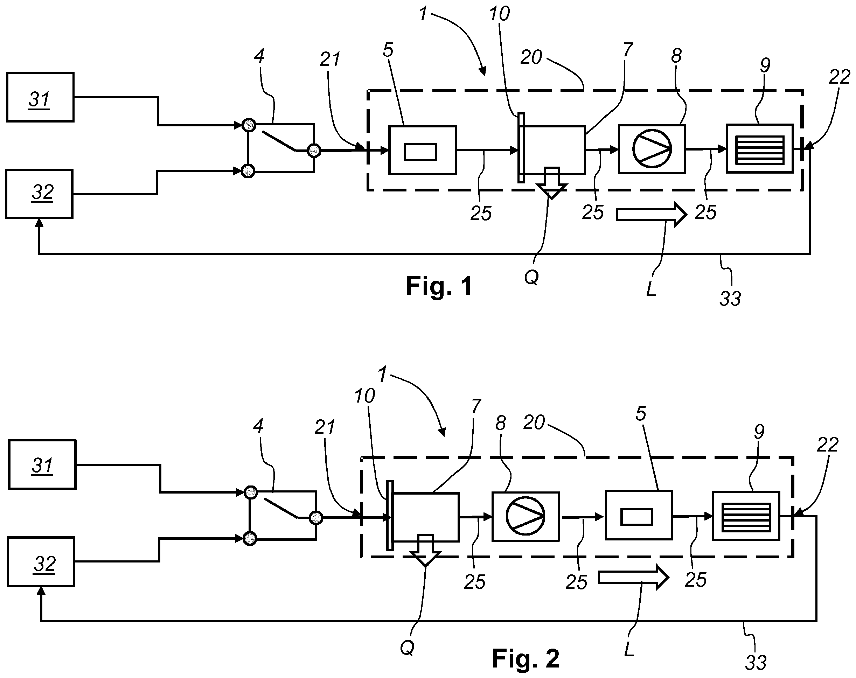

**Figur 1** zeigt schematisch den Aufbau eines Klimageräts 1, das bevorzugt für die Klimatisierung vom Fahrgastraum bzw. Innenraum von Kraftfahrzeugen verwendet wird. Das Klimagerät 1 besteht aus einem Gehäuse 20 (in den Figuren 1 bis 6 gestrichelt dargestellt). Das Gehäuse 20 hat eine Luftzufuhr 21 (für die nicht klimatisierte Luft) und eine Luftabfuhr 22 (klimatisierte und gereinigte Luft) ausgebildet. Zwischen der Luftzufuhr 21 und der Luftabfuhr 22 ist ein Strömungsweg 25 definiert. Im Strömungsweg 25 sind bei der in Figur 1 dargestellten Ausführungsform in Strömungsrichtung L im Gehäuse 20 nach der Luftzufuhr 21 eine Einrichtung 5 zur Erzeugung eines angeregten Gases, ein Wärmetauscher 7 und ein Gebläse 8 und eine Einrichtung 9 zur Steigerung der Aktivität/Reaktivität vorgesehen. Über den Wärmetauscher 7 wird der zu klimatisierenden Luft die Wärme Q entzogen und an die Umgebung abgeführt.

Gemäß einer Ausführungsform ist eine Oberflächenbeschichtung 10 beispielsweise am Wärmetauscher 7 vorgesehen. Die Oberflächenbeschichtung 10 dient zum katalytischen Abbau des Ozongehalts beziehungsweise des angeregten Gases bzw. Plasmas. Die Anbringung der Oberflächenbeschichtung 10 am Wärmetauscher 7 hat den Vorteil, dass hier eine große Oberfläche zur Verfügung steht, um den gewünschten Effekt des katalytischen Abbaus zu erreichen. Andere Möglichkeiten zum Vorsehen der Oberflächenbeschichtung 10 bieten das Gebläse 8 und/oder das Verdampfergebläse. Hier wird durch die hohen Turbulenzen der zu klimatisierenden Luft die ausreichende Wirkung für den katalytischen Abbau erreicht. Eine weitere Möglichkeit des Anbringens der Oberflächenbeschichtung 10 stellen die Innenwandungen (nicht dargestellt) des Verteilersystems 11 zur Verfügung. Dies hat den Vorteil, dass im Verteilersystem 11 ohnehin eine große Oberfläche zur Verfügung steht, ohne dass zusätzliche Bauteile für das Aufbringen der Oberflächenbeschichtung 10 erforderlich sind.

Der Luftzufuhr 21 des Gehäuses 20 ist ein Verteiler 4 vorgeordnet, so dass wahlweise dem Gehäuse 20 Umgebungsluft 31 oder Innenluft 32 zur Klimatisierung zugeführt werden kann. Von der Luftabfuhr 22 des Gehäuses 20 wird die klimatisierte Luft in den zu klimatisierenden Raumbereich 33 geleitet, der bei der in Figur 1 gezeigten Darstellung durch einen Pfeil zur Innenluft 32 dargestellt ist. Die Einrichtung 5 zur Erzeugung eines angeregten Gases ist ein PDD piezoelektrischer Transformator, der als ein Plasmaerzeuger, als lonisator oder als Ozongenerator wirkt. Durch das angeregte Gas erreicht man eine Entkeimung und eine Frischewirkung der zu klimatisierenden Luft.

**Figur 2** zeigt eine weitere Ausführungsform des erfindungsgemäßen Klimageräts 1. Hier ist eine andere Anordnung der die Klimatisierung bewirkenden Elemente des erfindungsgemäßen Klimageräts 1 innerhalb des Gehäuses 20 dargestellt. Auf die Luftzufuhr 21 folgt der Wärmetauscher 7, der mit der Oberflächenbeschichtung 10 versehen ist. In Strömungsrichtung L sind im Strömungsweg 25 dann weiter das Gebläse 8, die Einrichtung 5 zur Erzeugung eines angeregten Gases und die Einrichtung 9 zur Steigerung der Aktivität/Reaktivität angeordnet.

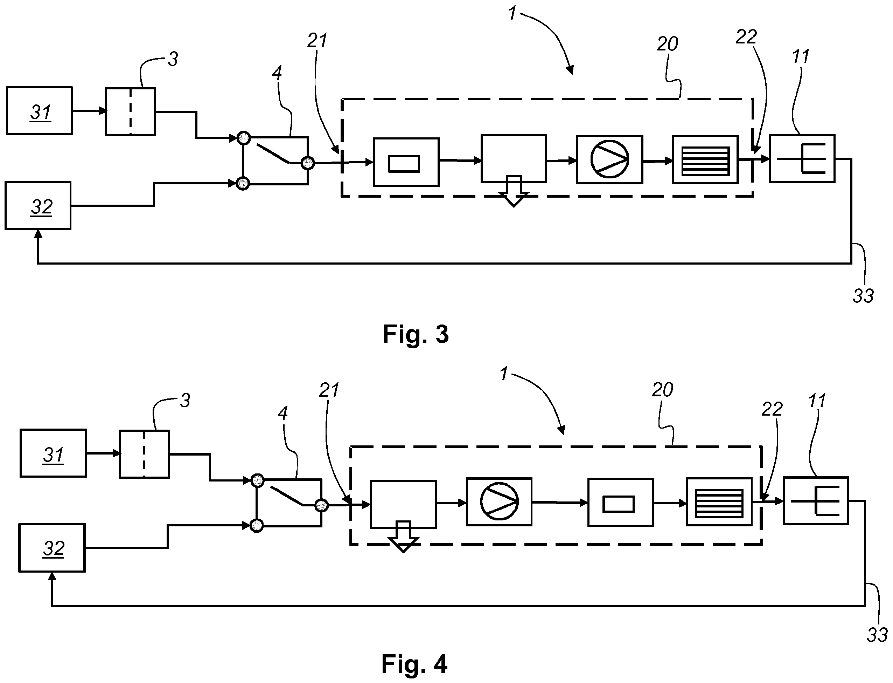

**Figur 3** und **Figur 4** zeigen Ausgestaltungen der Erfindung, wobei der Luftabfuhr 22 ein Verteilersystem 11 nachgeordnet ist. Mit dem Verteilersystem kann auf gezielte Weise die klimatisierte Luft in dem zu klimatisierenden Raumbereich 33 verteilt werden. Die Anordnung der Elemente im Gehäuse 20 der in Figur 3 beschriebenen Ausführungsform entspricht der Anordnung wie in Figur 1 beschrieben. Ebenso entspricht die Anordnung der Elemente im Gehäuse 20 der in Figur 4 beschriebenen Ausführungsform der Anordnung wie in Figur 2 beschrieben. Ebenso ist den in Figur 3 und Figur 4 beschriebenen Ausführungsformen eine Verteilerklappe 4, vor der Luftzufuhr 22 der Umgebungsluft 31, ein Filter 3 für Partikel, Schmutz oder Wasser vorgeordnet.

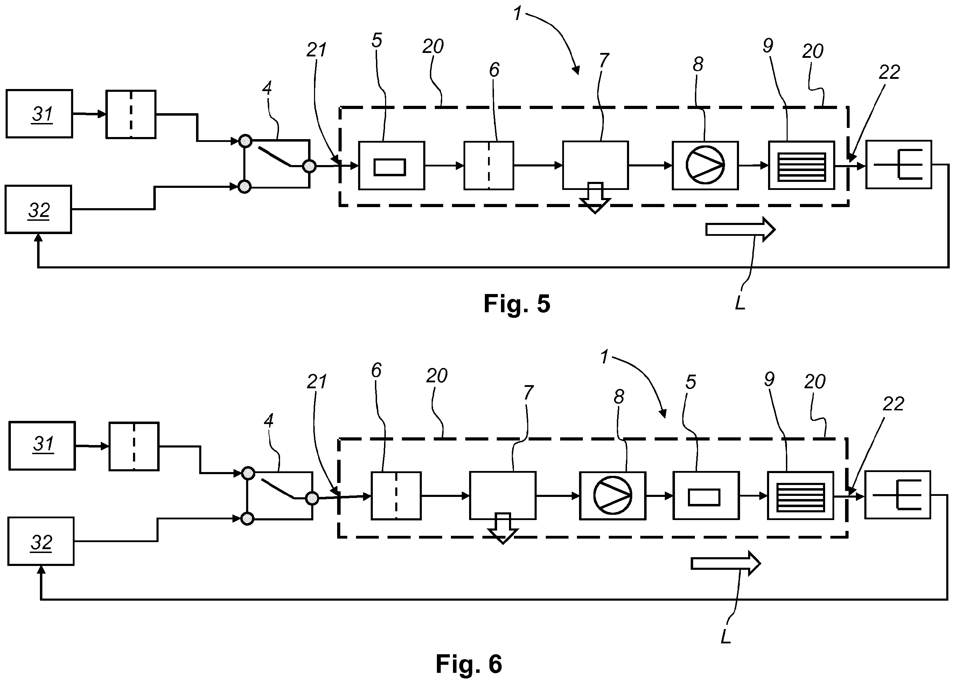

**Figur 5** zeigt eine weitere Ausführungsform des Klimageräts 1. Im Gehäuse 20 sind zwischen der Luftzufuhr 21 und der Luftabfuhr 22, in Strömungsrichtung L, die Einrichtung 5 zur Erzeugung eines angeregten Gases, ein Filter 6, der Wärmetauscher 7 (ggf. mit Oberflächenbeschichtung, hier nicht dargestellt), das Gebläse 8 und die Einrichtung 9 zur Steigerung der Aktivität/Reaktivität angeordnet. Der Filter 6 kann bereits reaktive Bestandteile oder katalytisch aktive Bestandteile enthalten, um den Ozongehalt der zu klimatisierenden Luft zu reduzieren.

**Figur 6** zeigt eine Abwandlung der in Figur 5 dargestellten Ausführungsform. Im Gehäuse 20 sind zwischen der Luftzufuhr 21 und der Luftabfuhr 22 in Strömungsrichtung L, der Filter 6, der Wärmetauscher 7 (ggf. mit Oberflächenbeschichtung, hier nicht dargestellt), das Gebläse 8, die Einrichtung 5 zur Erzeugung eines angeregten Gases und die Einrichtung 9 zur Steigerung der Aktivität/Reaktivität angeordnet.

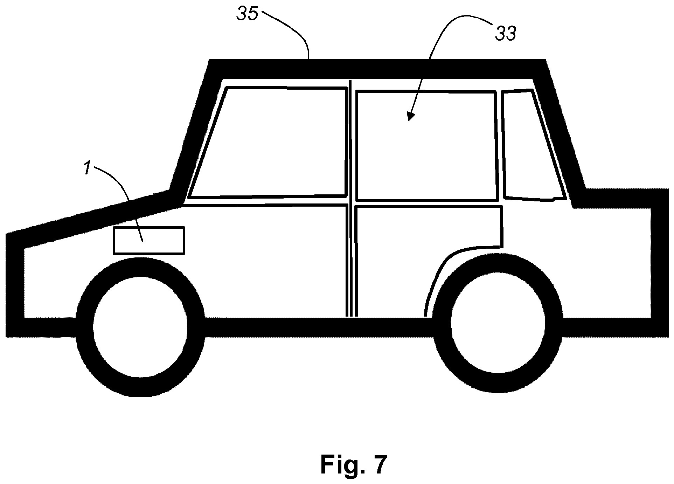

**Figur 7** zeigt eine schematische Darstellung der Verwendung des erfindungsgemäßen Klimageräts 1 bei einem Kraftfahrzeug 35 zur Klimatisierung. Der zu klimatisierende Raumbereich 33 ist der Innenraum bzw. die Fahrgastzelle des Kraftfahrzeugs 35.

Die Anmeldung wurde unter Bezugnahme auf bevorzugte Ausführungsformen beschrieben. Für einen Fachmann ist es jedoch vorstellbar, dass Abwandlungen oder Änderungen der Erfindung gemacht werden können, ohne dabei den Schutzbereich der nachstehenden Ansprüche zu verlassen. Alle zuvor beschriebenen Ausführungsformen des Klimageräts können sowohl einzeln als auch in jeder beliebigen Kombination Verwendung finden.

### Bezugszeichenliste:

- 1: Klimagerät
- 3: Filter
- 4: Verteiler
- 5: Einrichtung zur Erzeugung eines angeregten Gases
- 6: Filter
- 7: Wärmetauscher
- 8: Gebläse
- 9: Einrichtung zur Steigerung der Aktivität/Reaktivität
- 10: Oberflächenbeschichtung
- 11: Verteilersystem
- 20: Gehäuse
- 21: Luftzufuhr
- 22: Luftabfuhr
- 25: Strömungsweg
- 31: Umgebungsluft
- 32: Innenluft
- 33: klimatisierender Raumbereich
- 35: Kraftfahrzeug
- L: Strömungsrichtung
- Q: Wärme

## Patentansprüche

1. Klimagerät (1) für einen zu klimatisierenden Raumbereich (33), umfassend
ein Gehäuse (20) mit einer Luftzufuhr (21) und einer Luftabfuhr (22) und mindestens einem zwischen der Luftzufuhr (21) und der Luftabfuhr (22) ausgebildeten Strömungsweg (25), wobei im Strömungsweg (25) zumindest ein Wärmetauscher (7), ein Gebläse (8) und eine Einrichtung (5) zur Erzeugung eines angeregten Gases vorgesehen sind,
**gekennzeichnet dadurch, dass**
die Einrichtung (5) zur Erzeugung eines angeregten Gases ein Plasmaerzeuger, ein lonisator oder ein Ozongenerator ist und einen PDD piezoelektrischen Transformator umfasst.

2. Klimagerät (1) nach Anspruch 1, wobei im Strömungsweg (25) des angeregten Gases eine Oberflächenbeschichtung (10) zum katalytischen Abbau des Ozongehalts des angeregten Gases vorgesehen ist, die der Einrichtung (5) zur Erzeugung des angeregten Gases nachgeordnet ist.

3. Klimagerät (1) nach einem der vorangehenden Ansprüche, wobei eine Einrichtung (9) zur Steigerung der Aktivität/Reaktivität im Strömungsweg (25) im Gehäuse (20) angeordnet ist.

4. Klimagerät (1) nach Anspruch 3, wobei die Einrichtung (5) zur Erzeugung eines angeregten Gases zwischen dem Gebläse (8) und der Einrichtung (9) zur Steigerung der Aktivität/Reaktivität vorgesehen ist.

5. Klimagerät (1) nach Anspruch 3 oder 4, wobei das Gebläse (8) im Strömungsweg (25) der Luft der Einrichtung (9) zur Steigerung der Aktivität/Reaktivität vorgeordnet oder das Gebläse (8) in Strömungsrichtung (L) der Luft der Einrichtung (9) zur Steigerung der Aktivität/Reaktivität nachgeordnet ist.

6. Klimagerät (1) nach einem der vorangehenden Ansprüche, wobei innerhalb des Gehäuses (20) ein Filter (6) vorgesehen ist, der in Strömungsrichtung (L) der Luft dem Wärmetauscher (7) vorgeordnet ist.

7. Klimagerät (1) nach Anspruch 6, wobei die Einrichtung (5) zur Erzeugung eines angeregten Gases dem Filter (6) im Strömungsweg (25) der Luft vorgeordnet oder dem Filter (6) in Strömungsrichtung (L) der Luft nachgeordnet ist.

8. Klimagerät (1) nach einem der vorangehenden Ansprüche, wobei der Einrichtung (9) zur Steigerung der Aktivität/Reaktivität eine Katalysatorstruktur, eine Aktivkohlestruktur oder ein Reaktivfilter vorgeordnet ist.

9. Klimagerät (1) nach Anspruch 8, wobei die Einrichtung (9) zur Steigerung der Aktivität/Reaktivität eine katalytisch aktive Oberfläche aufweist, mittels der durch heterogene Oxidation Moleküle abbaubar sind.

10. Klimagerät (1) nach Anspruch 8, wobei die Einrichtung (9) zur Steigerung der Aktivität/Reaktivität eine katalytisch aktive Oberfläche aufweist, mittels der durch heterogene Oxidation Mikroorganismen im Wachstum gehemmt oder abgetötet werden.

11. Klimagerät (1) nach einem der vorangehenden Ansprüche, wobei der Luftzufuhr (21) in das Gehäuse (20) eine Verteilerklappe (4) vorgeordnet ist, die wahlweise mit einer Umgebungsluft (31) oder einer Innenluft (32) des zu klimatisierenden Raumbereichs (33) in fluider Verbindung ist.

12. Klimagerät (1) nach Anspruch 11, wobei der Verteilerklappe (4) vor der Zuführung der Umgebungsluft (31) ein Filter (3) für Partikel, Schmutz oder Wasser vorgeordnet ist.

13. Klimagerät (1) nach einem der vorangehenden Ansprüche, wobei der Luftabfuhr (22) aus dem Gehäuse (20) ein Verteilersystem (11) nachgeschaltet ist, so dass die klimatisierte Luft in gewünschter Weise im zu klimatisierenden Raumbereich (33) verteilbar ist.

14. Verwendung des Klimageräts (1) nach einem der vorangehenden Ansprüche zur Klimatisierung des Innenraums (33) eines Kraftfahrzeugs (35).

## Claims

1. Air-conditioning device (1) for a room area (33) to be air-conditioned, comprising a housing (20) having an air supply (21) and an air discharge (22) and at least one flow path (25) formed between the air supply (21) and the air discharge (22), wherein at least one heat exchanger (7), a fan (8) and a device (5) for generating an excited gas are provided in the flow path (25),
**characterized in that**
the device (5) for generating an excited gas is a plasma generator, an ionizer or an ozone generator and comprises a PDD piezoelectric transformer.

2. Air-conditioning device (1) according to claim 1, wherein in the flow path (25) of the excited gas, a surface coating (10) for catalytic degradation of the ozone content of the excited gas is provided, which is arranged downstream of the device (5) for generating an excited gas.

3. Air-conditioning device (1) according to one of the preceding claims, wherein a device (9) for increasing the activity/reactivity in the flow path (25) is arranged in the housing (20).

4. Air-conditioning device (1) according to claim 3, wherein the device (5) for generating an excited gas is provided between the fan (8) and the device (9) for increasing activity/reactivity.

5. Air-conditioning device (1) according to claim 3 or 4, wherein the blower (8) is provided in the flow path (25) of the air upstream of the device (9) for increasing activity/reactivity, or the blower (8) is provided in the flow direction (L) of the air downstream of the device (9) for increasing activity/reactivity.

6. Air-conditioning device (1) according to one of the preceding claims, wherein a filter (6) is provided inside the housing (20), which filter (6) is arranged upstream of the heat exchanger (7) in the flow direction (L) of the air.

7. Air-conditioning device (1) according to claim 6, wherein the device (5) for generating an excited gas is arranged upstream of the filter (6) in the flow path (25) of the air or is arranged downstream of the filter (6) in the flow direction (L) of the air.

8. Air-conditioning device (1) according to one of the preceding claims, wherein a catalyst structure, an activated carbon structure or a reactive filter is arranged upstream of the device (9) for increasing the activity/reactivity.

9. Air-conditioning device (1) according to claim 8, wherein the device (9) for increasing the activity/reactivity has a catalytically active surface by means of which molecules can be degraded by heterogeneous oxidation.

10. Air-conditioning device (1) according to claim 8, wherein the device (9) for increasing the activity/reactivity has a catalytically active surface by means of which microorganisms are inhibited in growth or killed by heterogeneous oxidation.

11. Air-conditioning device (1) according to one of the preceding claims, wherein a distributor flap (4) is arranged upstream of the air supply (21) into the housing (20), which distributor flap (4) is selectively in fluid connection with an ambient air (31) or an internal air (32) of the room area (33) to be air-conditioned.

12. Air-conditioning device (1) according to claim 11, wherein a filter (3) for particles, dirt or water is arranged upstream of the distribution flap (4) before the supply of the ambient air (31).

13. Air-conditioning device (1) according to one of the preceding claims, wherein a distribution system (11) is connected downstream of the air discharge (22) from the housing (20), so that the conditioned air can be distributed in a desired manner in the room area (33) to be conditioned.

14. Use of the air-conditioning unit (1) according to one of the preceding claims for air-conditioning the interior (33) of a motor vehicle (35).

## Revendications

1. Climatiseur (1) pour un volume (33) à climatiser, comprenant un boîtier (20) avec une arrivée d'air (21) et une sortie d'air (22) et avec au moins un trajet de circulation (25) entre l'arrivée d'air (21) et la sortie d'air (22), dans lequel sont prévus dans le trajet de circulation (25) au moins un échangeur de chaleur (7), une soufflerie (8) et une installation (5) destinée à produire un gaz excité,
**caractérisé en ce que** l'installation (5) destinée à produire un gaz excité est un générateur de plasma, un ioniseur ou un générateur d'ozone et comprend un transformateur piézoélectrique PDD.

2. Climatiseur (1) selon la revendication 1, dans lequel est prévu dans le trajet de circulation (25) du gaz excité un revêtement de surface (10) pour la dégradation catalytique de l'ozone contenu dans le gaz excité, qui est monté en aval de l'installation (5) destinée à produire un gaz excité.

3. Climatiseur (1) selon l'une des revendications précédentes, dans lequel une installation (9) destinée à accroître l'activité/la réactivité dans le trajet de circulation (25) est disposé dans le boîtier (20).

4. Climatiseur (1) selon la revendication 3, dans lequel l'installation (5) destinée à produire un gaz excité est prévue entre la soufflerie (8) et l'installation (9) destinée à accroître l'activité/la réactivité.

5. Climatiseur (1) selon la revendication 3 ou 4, dans lequel la soufflerie (8) est disposée, dans le trajet de circulation (25) de l'air, en amont de l'installation (9) destinée à accroître l'activité/la réactivité ou la soufflerie (8) est disposée, dans le sens de circulation (L) de l'air, en aval de l'installation (9) destinée à accroître l'activité/la réactivité.

6. Climatiseur (1) selon l'une des revendications précédentes, dans lequel est prévu à l'intérieur du boîtier (20) un filtre (6) qui est monté en amont de l'échangeur de chaleur (7) dans le sens de circulation (L) de l'air.

7. Climatiseur (1) selon la revendication 6, dans lequel l'installation (5) destinée à produire un gaz excité est montée en amont du filtre (6) dans le trajet de circulation (25) de l'air ou montée en aval du filtre (6) dans le sens de circulation (L) de l'air.

8. Climatiseur (1) selon l'une des revendications précédentes, dans lequel une structure de catalyseur, une structure au charbon actif ou un filtre réactif est montée en amont de l'installation (9) destinée à accroître l'activité/la réactivité.

9. Climatiseur (1) selon la revendication 8, dans lequel l'installation (9) destinée à accroître l'activité/la réactivité présente une surface catalytique active au moyen de laquelle des molécules peuvent être dégradées par oxydation hétérogène.

10. Climatiseur (1) selon la revendication 8, dans lequel l'installation (9) destinée à accroître l'activité/la réactivité présente une surface catalytique active au moyen de laquelle des micro-organismes sont empêchés de se multiplier ou éliminés par oxydation hétérogène.

11. Climatiseur (1) selon l'une des revendications précédentes, dans lequel est monté en amont de l'arrivée d'air (21) dans le boîtier (20) un clapet de distribution (4) qui peut être mis en communication pour la circulation de fluide soit avec l'air ambiant (31), soit avec l'air intérieur (32) du volume (33) à climatiser.

12. Climatiseur (1) selon la revendication 11, dans lequel un filtre (3) pour les particules, la saleté ou l'eau est monté en amont du clapet de distribution (4) avant l'arrivée de l'air ambiant (31).

13. Climatiseur (1) selon l'une des revendications précédentes, dans lequel un système de distributeur (11) est monté en amont de la sortie d'air (22) hors du boîtier (20), de sorte que l'air climatisé peut être distribué de la façon souhaitée dans le volume (33) à climatiser.

14. Utilisation du climatiseur (1) selon l'une des revendications précédentes pour climatiser l'intérieur (33) d'un véhicule à moteur (35).
